# EUROPEAN PATENT SPECIFICATION

(11) **EP 1 429 661 B1**
(45) Date of publication and mention of the grant of the patent: **06.01.2010**
(21) Application number: 02779315.7
(22) Date of filing: 05.09.2002
(51) Int. Cl.: A61B 17/00

(54) **APPARATUS FOR SEALING A BODY VESSEL PUNCTURE**
GERÄT FÜR DEN VERSCHLUSS EINER PUNKTIONSSTELLE IN EINEM KÖRPERGEFÄSS
DISPOSITIF POUR L'OBTURATION DE VAISSEAU CORPOREL PERFORE

(30) Priority: 07.09.2001 SE 0102968
(43) Date of publication of application: 23.06.2004
(73) Proprietor: Abbott Laboratories Vascular Enterprises Limited, Terrace, Dublin 2 (IE)
(72) Inventor: SOLEM, Jan, Otto, 8234 Stetten (CH)
(74) Representative: Åkesson, Sten Jan-Åke
(86) International application number: PCT/EP2002/009962
(87) International publication number: WO 2003/022158

(56) References cited:
- WO-A-98/31286
- NL-A- 9 302 140
- US-A- 5 916 236
- US-A- 6 126 675

## Description

### Technical Field of the Invention

The present invention is generally related to the sealing of a puncture or an incision in a body vessel, e.g. a blood vessel, bile duct, urinary duct or gastrointestinal tract, and more specifically to an apparatus for performing such a sealing.

### Background of the Invention

Heart diseases, and coronary artery disease in particular, are the most common cause of death. Diagnostic cardiac catheterization and interventions by means of catheters against vascular and heart diseases are today one of the most common procedures in medical practice.

In order to access the vascular system, needles are advanced through the skin and into the vessels. The holes thereby created in the vessels (referred to as "punctures" in the following as a generic term for punctures and incisions) are thereafter dilated to a desired size for catheters to be inserted and used for treatment. The channels thereby formed are usually from 2.5 mm to 5 mm in diameter, and larger channels have to be closed by surgical intervention. The smaller and the most common channels are left to close by nature. The latter means that a clot has to be formed in the channels by blood platelets and the coagulation factors from the blood itself. To allow this process to take place, compression from the outside of the skin is mandatory for hours.

Despite this cumbersome and time consuming procedure, many patients develop collections of blood under the skin, so called hematomas, which in many cases can become of considerable size. As a matter of fact, many patients need blood transfusions and sometimes even surgery is needed. Other patients suffer discomfort from smaller hematomas. Such problems may become really troublesome for patients who are under anticoagulation treatment, as a good number of heart patients are.

Most known sealing methods and devices include some type of suturing that makes them complicated, time consuming and difficult to use.

US-A-4,744,364 discloses a device for sealing a puncture in the wall of a lumen of a living being, including an elongated tubular body having an outlet at the distal end thereof and which is arranged to be inserted through the puncture so that the outlet is located within the vessel. An expandable closure is disposed within the tubular body and is formed so as to be held in a compact configuration therein. The tubular body also includes ejecting means for forcing the closure out of the outlet into the interior of the blood vessel, whereupon the closure automatically expands to form an enlarged engagement surface. A retraction filament is secured to the closure for pulling it to the puncture after the tubular body has been withdrawn from the puncture so that the engagement surface of the closure hemostatically engages the inner surface of the blood vessel contiguous with the puncture. The filament is held taut and taped or otherwise secured in place on the patient's skin to hold the closure in position. The closure and filament are each formed of a biodegradable material to enable the closure to be left in place.

As described in the prior-art section in US 5,916,236, the arrangement in the '364 patent presents a drawback relating to uncontrolled tension in the retraction filament due to the dissolving material and movements of the filament which is attached to the outside skin.

US-A-5,413,571 discloses a device and a method for sealing punctures and incisions without the use of suturing. The device includes a biodegradable, fluid-retaining sealing balloon presenting a distal end. In use, the device is inserted into the puncture, where after the distal balloon end is inflated by a fluid (such as a saline solution) retained inside the balloon. The thus expanded balloon end is brought into contact with the vessel in order to seal the puncture. The device further includes a shaft member left to be resorbed inside the body. The shaft member may be sutured to the skin or slightly below the outer surface of the skin.

US-A-5,916,236 mentioned above discloses another device for sealing a puncture, comprising a retaining element having a distal occlusion element. In use, the retaining element and the occlusion element are fixed in the puncture by a fixing element positioned outside the vessel. The three elements are left in the body to be resorbed.

A specific drawback of the above and other prior-art devices and methods for sealing a body vessel puncture of incision is the fact that they may be incorrectly positioned and incorrectly sealed.

A sealing apparatus as defined in the preamble of claim 1 is disclosed in US-A-6 126 675.

### Summary of the Invention

An object of the present invention is to provide an improved apparatus for sealing a body vessel puncture that is simple to use and which allows the sealing to be performed in an efficient and safe manner.

A specific object of the invention is to provide an apparatus that makes it possible to avoid incorrect positioning of the apparatus as well as incorrect sealing of the puncture.

Another object of the invention is to provide an apparatus that makes it possible to avoid the requirement of using suturing when performing the sealing procedure.

Another object of the invention is to provide a solution that makes it possible to reduce the need for observation of the patient after treatment and thereby make the patient mobile within a short time period.

Having regard to the above and other objects, there is provided in accordance with the invention an apparatus as defined in claim 1. Preferred embodiments thereof are set out in the dependent claims.

According to the invention, there is provided an apparatus for sealing a puncture in a body vessel, said apparatus including:
a sealing member comprising a flexible tubing having an internal passageway and being provided at an open distal end thereof with an elastic expansion member for self-expanding said open distal end to an expanded configuration when said elastic expansion member has been introduced into the vessel, such that the tubing at the distal open end thereafter may be brought into sealing contact with an inner wall of the vessel; and
a pursing member which is arranged to purse said flexible tubing in order to seal said passageway.

One advantage of the apparatus according to the invention is that it makes it possible for the user to obtain a visible indication of a correct positioning of the apparatus as well as the establishment of a correct sealing. More specifically, a body fluid flow, especially a blood flow, through the open distal end and through the internal passageway of the flexible tubing gives the user a visible indication that the expanded, distal open end of the tubing has been positioned inside the body vessel. After receiving such an indication, the user may apply said means for pursing the flexible tubing in order to seal the internal passageway thereof and restrict further blood flow through the passageway. Again, when the blood flow is discontinued through the passageway this gives the user a visible indication that the pursing of the tubing, i.e. the sealing of the passageway, is correct.

Another advantage of the apparatus according to the invention is that it makes it possible to obtain an efficient seal between the flexible tubing and the body vessel in a simple but still secure manner. More specifically, this sealing may be provided as a combined effect of the expanded elastic member and the applied pursing member. The pursing member, when occupying its final position outside the body vessel in which the pursing member purses the flexible tubing and thereby seals said passageway, will maintain the distal end part of the flexible tubing in a funnel-shaped form, since the actual end opening of the flexible tubing may be kept expanded by means of the elastic member, wheres the flexible tubing at a position outside the body vessel may be kept pursed by the pursing member. If the pursing member and the flexible tubing are designed such that the pursing member not only performs its pursing effect, but also a limited displacement-restricting effect on the tubing, the latter will be kept in position by the pursing member. The result of this is that the distal region of the flexible tubing will achieve a funnel shape when the pursing member is brought into position, whereby an inwardly and upwardly directed force in the wall of the tubing is created. Due to the elastic, outwardly directed force of the expansion member, the inwardly directed force acting on the tubing wall will not make the tubing collapse. However, due to the upwardly directed force the distal expanded end of the tubing will be held or pressed sealingly against the inner wall of the body vessel.

In order to restrict movement not only of the pursed tubing in relation to the pursing member, but also movement of the pursing member in relation to the body, the pursing member may be designed with projections or other means arranged to restrict or counteract unintentional movement of the pursing member. However, such movement-restriction means may preferably be designed such that it is still possible to manually displace the pursing member along the tubing in order to position the pursing member in its final position outside the body vessel.

It should be noted that the pursing member may be designed to provide but a "local" pursing of the flexible tubing, i.e. over a limited portion of the length of the tubing. Thus, the tubing will normally not be pursed along a major part and at least not along the distal part which is to be funnel-shaped. Using a pursing member having a limited extension in the direction of the tubing may also be advantageous for maintaining the pursing member in position in relation to the tissue.

In situations where the invention is used in connection with performing the puncture through the skin of the patient, such as in connection with catheterization, the flexible tubing preferably presents a sufficient length for the tubing, when positioned in the body, to extend from said puncture to the outside of the body. Selecting a sufficient length for the tubing not only allows the user to use the tubing for applying a light traction for localizing the distal open end correctly inside the body vessel. A sufficient length of the tubing and, thereby, the internal passageway thereof, also provides for said indicative blood flow. Thereby, the blood-flow indication becomes visible outside the patient. Thirdly, a sufficient length of the flexible tubing also makes it possible, as will be described below, to mount or arrange the pursing member on the flexible tubing not until after the latter has been introduced into the patient.

However, the invention may also be used in connection with open surgery. In such cases it may be of less importance to use any specific length of the flexible tubing.

In a preferred embodiment, the flexible tubing may be flexible in the sense that it has essentially no ability to maintain its shape by its own. This may be advantageous not only with respect to the introduction, but also the final healing result since the tubing as such will not essentially deform the surrounding tissue. The flexible tubing may be flexible along its longitudinal axis as well as along its radial axis. This may increase the pressing force generated when applying the pursing member, whereby the expansion member is even tighter pressed against the inner wall of the body vessel providing an even more secure sealing of the expansion member to the inner wall of the body vessel.

The elastic member, which is self-expandable to an expanded configuration, may be implemented in different ways. However, it is preferred that it is designed in such a way that it does not block fluid flow from the vessel and out through the expanded open distal end of the flexible tubing.

Preferably, the elastic member is flexible in the sense that it can adapt its shape to the curvature of the inner wall of the body vessel at the site of the puncture.

In a preferred embodiment, the elastic member is ring-shaped and arranged circumferentially at the distal end opening of the flexible tubing. Thereby, the elastic member may provide a "sealing rim" to be brought into sealing contact with the inner wall of the body vessel, preferably due to the pressing force generated when applying the pursing element.

In a preferred embodiment, the elastic member is foldable to a non-expanded configuration, allowing said elastic member to be introduced through the puncture, and unfoldable in a self-expandable manner to said expanded configuration.

Preferably, the elastic member is integrally formed with the flexible tubing at the distal open end thereof. It may be integrally formed in the form of a reinforcement of the flexible tubing. It may be implemented in the form of a material thickening of said tubing and/or in the form of a stiffening means attached to said tubing.

The elastic member may include an X-ray absorbing material for confirming the correct positioning thereof by X-ray examination.

The pursing member may be formed as a member separate from said flexible tubing. The term "pursing member" should be interpreted as a member that, when positioned on the flexible tubing, is able to purse the flexible tubing to such an extent that the internal passageway thereof is substantially sealed. This may be accomplished by a ring-shaped member that is positioned around the flexible tubing (from the outset or after the tubing has been inserted) and displaced to its final pursing-position. In this case, the "pursing effect" is primarily due to a restricted central opening in ring-shaped member, i.e. a geometrical effect. The ring-shaped pursing member may also be elastic and striving to closing its central opening, whereby the "pursing effect" is further enhanced by an elastic force. A ring-shaped pursing member may have an articulation and a lock allowing it to be positioned from the side relative to the flexible tubing. However, the "pursing effect" may also be accomplished by a clip, generating a clamping force around the flexible tubing.

The sealing member, i.e. the flexible tubing with its elastic member, may include a material selected from a group consisting of a synthetic medical plastic material like polyurethane, polyvinyl, polytetrafluorethylene (PTFE) or other polymers, and a biologically degradable material like polydioxanone (PDS), polyglactin (Vicryl), polyglycolic acid (Dexon) or other resorbable materials that slowly will be absorbed by the body.

The pursing or clamping member may include the same materials as above.

In a preferred embodiment, the apparatus according to the invention further includes an insertion tube having a central bore or channel, which is arranged to initially receive the elastic member and at least part of the flexible tubing, thereby forming a three-part assembly to be inserted as a unit towards the puncture. The insertion tube serves to maintain the elastic member in a non-expanded configuration, e.g. a folded configuration, during the insertion procedure. The insertion tube also facilitates the insertion of flexible tubing. The insertion tube may be pliable.

Preferably, such an insertion tube with the non-expanded elastic member therein is advanced through a therapeutic sheath that is left in the puncture after the intervention, thus also serving to guide the insertion tube towards the puncture. The distal end of the insertion tube is advanced all the way through the puncture and into the vessel. The elastic member may then self-expand to its expanded configuration. Thereafter, the outer sheath may be removed.

In the embodiment wherein such a insertion tube is used, the positioning of the sealing member inside the insertion tube is preferably performed by a manufacturer, or at least before the actual use of the apparatus.

A pushing device, such as a solid rod or a tube may be used for the insertion procedure described above. The pushing device may also be used for pushing the compressed or folded elastic member out from the distal end of the insertion tube.

According to another aspect not forming part of the the invention, there is further provided a method for sealing a puncture in a body vessel, comprising:
introducing a distal end of a sealing member through the puncture, said sealing member comprising a flexible tubing having an internal passageway and an elastic expansion member at its distal end,
allowing the elastic expansion member to self-expand to an expanded configuration in the vessel,
detecting a blood flow through the internal passageway as an indication of the distal end of the sealing member being positioned inside the body vessel, and thereafter
sealing the internal passageway by pursing the flexible tubing outside the body vessel.

An advantage of this method is that it provides a possibility to control the positioning of the sealing member during the procedure. When a blood flow through the internal passageway of the tubing is observed or detected by the eye or by some other means, this constitutes an indication of the distal end of the sealing member being positioned correctly inside the vessel to be sealed. Thereafter, when the tubing is pursed, the blood flow is stopped. Again, an indication is received that the puncture has been successfully sealed.

According to another aspect not forming part of the invention, there is provided a solution that is primarily directed to solve the sealing problem in an efficient but yet simple manner. According to this aspect there is provided an apparatus for sealing a puncture in a body vessel, said apparatus including:
a sealing member comprising a flexible tubing being provided at a distal end thereof with an elastic expansion member for self-expanding the distal end to an expanded configuration when said elastic expansion means have been introduced through the puncture and into the vessel,
such that the tubing at the distal end thereafter may be brought into sealing contact with an inner side of the vessel circumferentially of said puncture; and
a pursing member which is arranged to purse said flexible tubing circumferentially outside the body vessel, said distal end of the tubing thereby being formed to a funnel-shaped sealing against an inner wall of the body vessel.

According to this second aspect, the pursing member, when positioned in its final pursing position, will generate a tension or tensile stress in the distal funnel-shaped part of the tubing wall, which in its turn will press the distal region of the tubing into a sealing engagement with the inner wall of the body vessel. Although it may in some circumstances be advantageous to also use the visible blood-flow indication described above, this sealing effect obtained by the combination of the elastic member and the pursing member may be used as a separate effect also in embodiments presenting no indicative blood flow through the tubing. In other words, it may be possible to apply this sealing technique to embodiments presenting no open distal end of the flexible tubing.

Further, the pursing member may be initially disposed on the tubing already before the use of the, apparatus is initiated. The pursing member may then initially be in a proximal position on the tubing and during the sealing of the puncture be brought to a distal position in which the flexible tubing is pursed to form the funnel-shaped sealing. A side-hole in the flexible tubing may be located distal of the initial position of the pursing member, again allowing a blood flow through the flexible tubing to indicate the position of the distal end of the sealing member before the pursing member is advanced to its final position.

According to this second aspect not forming part of the invention, there is further provided a method for sealing a puncture in a body vessel, comprising:
introducing a distal end of a sealing member through the puncture, said sealing member comprising a flexible tubing having an elastic expansion member at its distal end,
allowing the elastic expansion member to self-expand to an expanded configuration in the vessel, and
pursing the flexible tubing outside the body vessel such that the distal end of the sealing member obtains a funnel-shape and seals against the inner wall of the body vessel.

### Brief Description of the Drawings

By way of example, the invention will now be described in more detail with reference to the accompanying drawings, showing a presently preferred embodiment of an apparatus as well as a method, the latter not forming part of the invention.

Fig. 1 schematically illustrates three parts included in an embodiment of a sealing apparatus according to the present invention.

Fig. 2 shows an insertion assembly comprising a sealing member and an insertion tube .

Figs 3-10 schematically illustrate the use of the sealing apparatus of Figs 1-2 for sealing a puncture in a body vessel.

### Detailed Description of a Preferred Embodiment

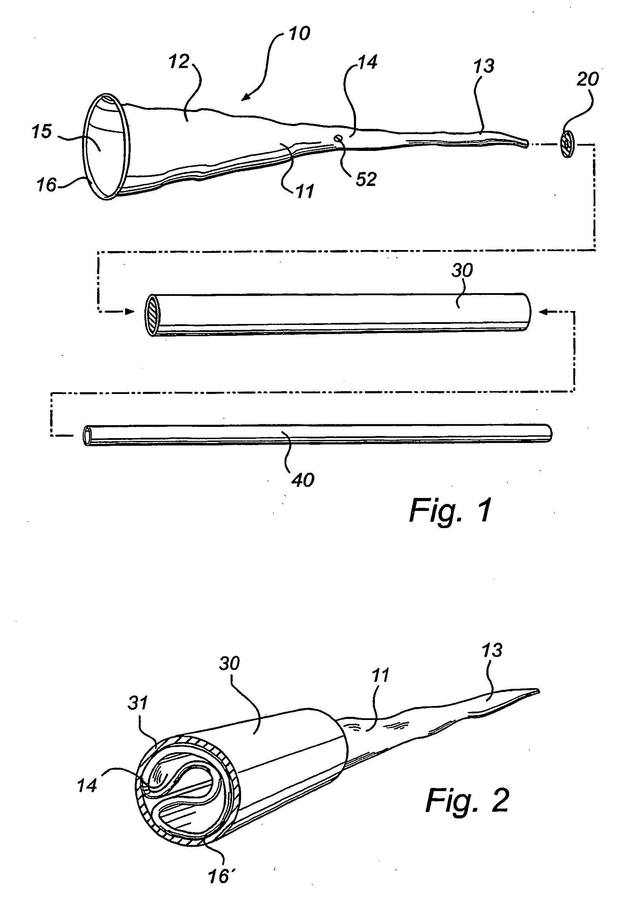

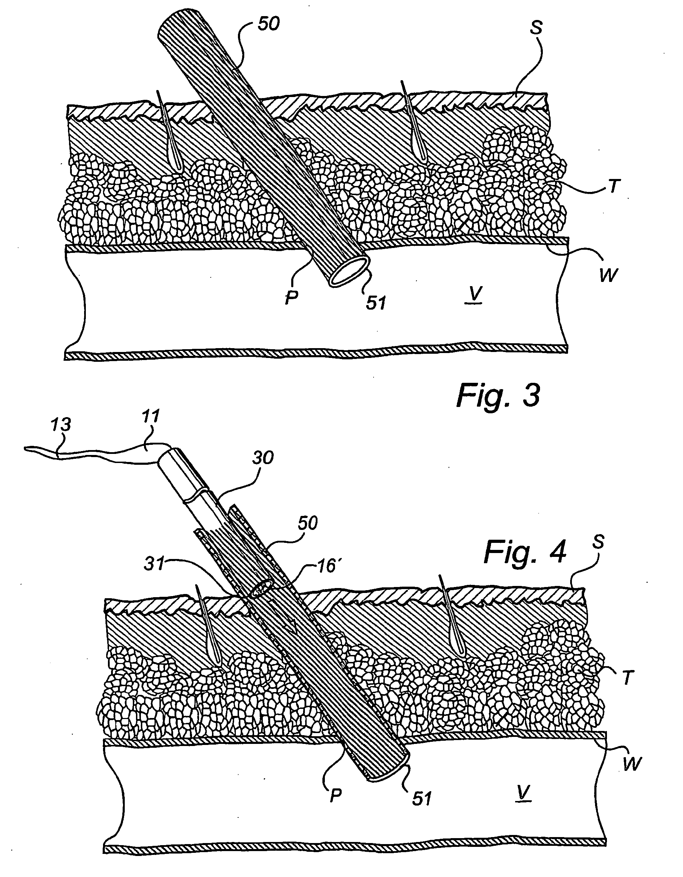

Fig. 1 discloses an embodiment of an apparatus according to the invention for sealing a puncture P in a body vessel V (see Fig. 3), such as an artery. The disclosed embodiment of the sealing apparatus may be said to comprise four main parts: a sealing member 10, a pursing member 20, an insertion tube 30 and a pushing device 40.

In the disclosed embodiment, the sealing member 10 comprises an elongated, flexible, thin-walled tubing 11, wherein the terms "flexible and thin-walled" should be construed as meaning that the wall of the tubing 11 has essentially no ability to maintain its shape by its own. From a use point of view, the tubing 11 presents a distal end region 12 and a proximal region 13. Furthermore, the tubing 11 defines an internal passageway 14 leading from a distal end opening 15 towards the proximal region 13.

The tubing 11 may have an open proximal end allowing blood flowing through the internal passageway 14 to exit the tubing 11. Alternatively, a blood-receiving compartment (not shown) may be arranged at the proximal end for collecting blood flowing through the internal passageway 14.

The flexible tubing 11 may present an essentially constant cross section along its length or, as an alternative, the tubing 11 may be either straight or tapering proximally from its distal end region, whereby the distal end has the largest diameter of the tubing 11 (as shown in Fig. 1). A combination thereof is also possible. The preferred cross-sectional shape of the tubing 11 is circular, but other cross-sectional shapes are possible. Preferably, the diameter of the tubing 11 in the distal region is essentially larger than the width of the puncture. Preferably, the tubing 11 has a length at least extending from the vessel to the outer surface of the skin.

The tubing 11 is provided with an elastic member 16 at its distal end region 12. In this embodiment, the elastic element 16 is arranged circumferentially at the periphery of the distal end opening 15. The primary purpose of the elastic element 16 is to expand the distal end region 12 of the tubing 11 to a size greater than the dimension of the puncture to be sealed.

In this embodiment the elastic element 16 is implemented as an elastic reinforcement. Other embodiments are possible in which the elastic member 16 not necessarily presents any "reinforcement effect", but rather serves only to perform the self-expansion effect and, thereby, to counteract a complete retraction of the sealing member 10 out from the body vessel V. However, in the description of the present embodiment and the use thereof, the elastic member 16 will be referred to as "the reinforcement".

The reinforcement 16 is preferably designed such that it does not block the distal end opening 15 and the internal passageway 14 of the tubing 11, in order not to block an indicative blood flow.

The reinforcement 16 is preferably a local material thickening, but it may also be implemented as a stiffening integrated in the wall of the tubing.

The reinforcement 16 may be brought into a non-expanded configuration, i.e. a "compressed state", to such an extent that it may then be introduced into the body vessel through the puncture. In the present embodiment, the reinforcement 16 is able to be compressed inside the insertion tube 30. When the compression is relieved the reinforcement 16 essentially immediately reverts by a self-expansion action to its initial expanded configuration, i.e. the ring-shape in this embodiment. Thereby, the distal end region 12 of the tubing 11 will also expand.

The diameter of the tubing 11 at its distal end is essentially equal to the diameter of the expanded reinforcement 16. Preferably, the diameter of the tubing 11 at its distal end is slightly smaller than the diameter of the expanded reinforcement 16. Thus, a tension is created in the tubing wall when the reinforcement 16 is expanded.

The reinforcement 16 is preferably also flexible enough to be able to adjust to the curvature of the inner wall W of the body vessel V. The preferred shape of the reinforcement 16 is circular but other shapes are possible.

Further, the reinforcement 16 may include an X-ray absorbing material making the reinforcement 16 visible on the X-ray display during fluoroscopy and X-ray examination. Such material may be the flexible memory metal Nitinol, i.e. an alloy of nickel and titanium mainly. An X-ray check would then be possible to confirm the correct positioning of the reinforcement 16.

The material in the sealing member 10 may be a synthetic, medical plastic material like polyurethane, polyvinyl, polytetrafluoroethylene (PTFE) or other polymers. It may also be made of biologically degradable materials like polydioxanone (PDS), polyglactin (Vicryl), polyglycolic acid (Dexon) or other resorbable materials that slowly is resorbed by the body. The material in the sealing member 10 may also be any combination of these materials.

In the disclosed embodiment, the sealing member 10 is used together with the insertion tube 30. However, in a simpler embodiment the inventive sealing operation may be performed also without the use of the insertion tube 30. The insertion tube 30 is preferably a pliable tube having a diameter smaller than a therapeutic sheath 50 that is used in the body vessel V. Preferably, the sealing member 10 - at least the reinforcement 16 thereof - is delivered from the manufacturer in a compressed or folded state 16' inside the insertion tube 30, as illustrated in Fig. 2.

The positioning of the sealing member 10 inside the insertion tube 30 is preferably made by the manufacturer in order to maintain the sterility and reduce manual handling.

When the insertion tube 30 is inserted into the sheath 50, the sealing member 10 may be pushed into the body vessel V by the pushing device 40. This step will be described in detail further on.

The diameter or width of the pushing device 40 should thus be smaller than the internal diameter of the insertion tube 30 and the length should be longer than that of the insertion tube 30. As an example, the pushing device 40 may be in the form of a solid rod or a tube. In order to follow the curvature of the insertion tube 30 and the therapeutic sheath 50 the pushing device 40 is preferably flexible in such an extent that it may follow this curvature.

When the sealing member 10 has been positioned in the body vessel V it is pursed by the pursing member 20, preferably but not necessarily as close to the outer side of the body vessel V as possible. The pursing makes the distal region 12 of the sealing member 10 form a funnel-shape, pressing the reinforcement 16 against the inner wall W of the body vessel V to provide a sealing around the puncture P. Correctly positioned, the pursing member 20 lies adjacent the body vessel V.

The pursing member 20 is preferably a ring as shown in Fig. 1 that can be applied around the tubing 11 from the outside of the patient's body and then being advanced through the skin S along the tubing 11 towards the body vessel V. When used during open surgery, the pursing member may be a clip (not shown), and in this case the tubing 11 need not have any specific length.

The pursing member 20 may be initially disposed on the tubing 11 already when the apparatus is manufactured. The pursing member 20 may then initially be in a proximal position on the tubing 11 and during the sealing of the puncture be brought to a distal position in which the flexible tubing 11 is pursed. In this case, a side-hole 52 may be arranged in the flexible tubing 11 distal of the initial position of the pursing member 20 for allowing blood flow through the internal passageway 14 to exit the tubing 11.

The material in the pursing member 20 is preferably a material that can be resorbed by the body, e.g. the same as the material in the sealing member 10.

The parts included in the described embodiment may have a very simple design and are easy to produce. They are preferably delivered as a set contained in a sterile ready-to-use package. After use, all parts but the sealing member 10 and the pursing member 20 are disposed.

Referring now to Figs 3-10, the method of using the sealing apparatus in Figs 1 and 2 will be described.

In Fig. 3 a therapeutic sheath 50 has been advanced with a distal region 51 thereof through the skin S and into an artery V making a puncture P. Having completed the intervention, such as a catheterization, for which purpose the puncture have been made, the puncture is to be sealed. The insertion tube 30 containing the sealing member 10 with the compressed or folded reinforcement 16' therein is advanced through the therapeutic sheath 50 that is left in the puncture P of the artery V after the intervention, as illustrated in Fig. 4. Preferably, but not necessarily, the insertion tube 30 is longer than the sheath 50, such that the insertion tube 30 can be operated manually in all positions.

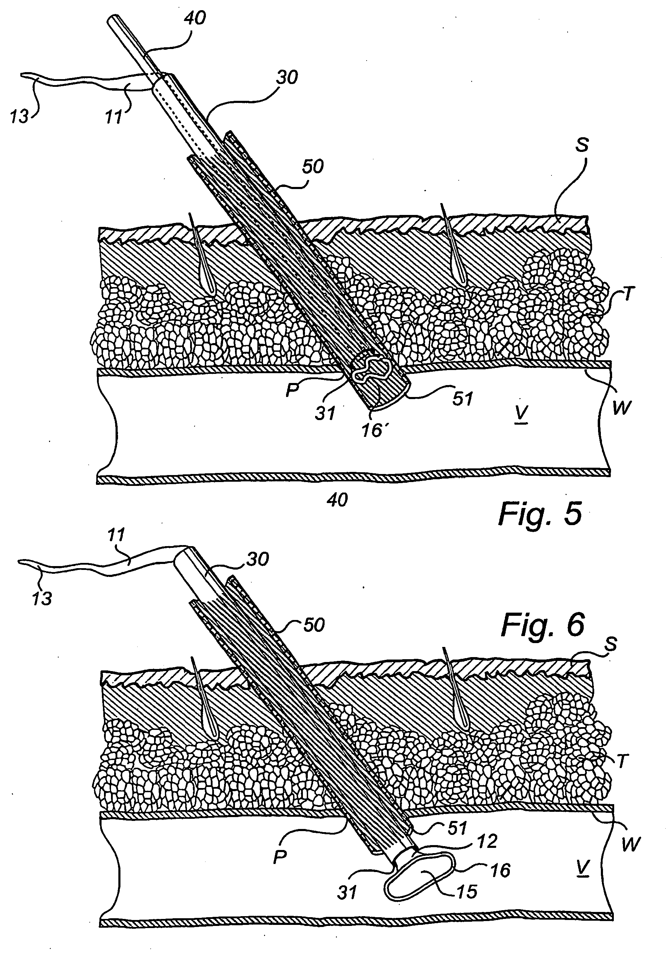

By means of the pushing device 40 the folded reinforcement 16 is pushed out of the distal end 31 of the insertion tube 30. This may be done while the reinforcement 16 is still present inside the sheath 50 at a distal region 51 thereof, as shown in Fig. 5. As an alternative, the distal end 31 of the insertion tube 30 may be introduced all the way into the vessel V, beyond the distal end 51 of the sheath 50, where after the reinforcement 16 is pushed out from the insertion tubing. 30.

Thus, in the present embodiment the reinforcement 16 unfolds inside the sheath 50 to the size of the inner diameter of the sheath 50, as shown in Fig. 5. A temporary sealing effect and, thereby, a reduced blood flow out through the sheath 50, may be obtained at this stage, possibly giving an indication to the user that the reinforcement 16 has been pushed out from the insertion tube 30.

Now, from the situation in Fig. 5 the distal end 31 of the insertion tube 30 and the not yet completely unfolded reinforcement 16 are further advanced all the way through the puncture P and into the artery V, as shown in Fig. 6.

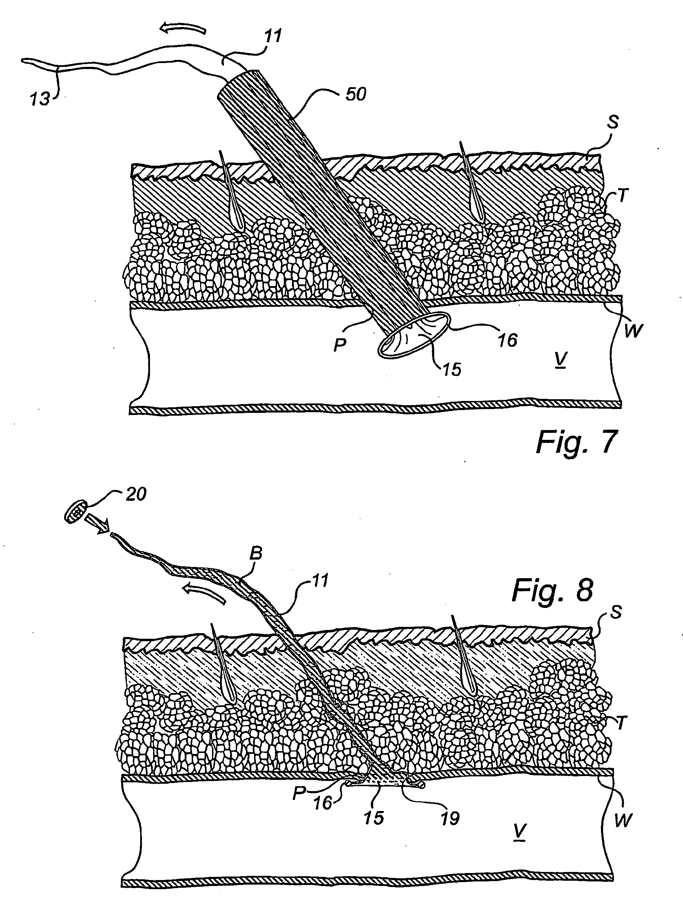

As shown in Fig. 7, after having removed the insertion tube 30, a light traction is applied to the tubing 11 letting the reinforced end region 12 thereof localize itself around the distal tip 51 of the sheath 50. A definite flow.of blood B as indicated with an arrow will now be present through the distal open end 15 and the internal passageway 14 of the sealing member 10, indicating to the user that the reinforced, distal end region 12 of the tubing 10 has been expanded correctly inside the artery V.

In case the pursing member 20 is initially disposed on the tubing 11, the flow of blood B will be present through the side-hole 52 in the tubing 11.

Simultaneously, blood flow through the therapeutic sheath 50 or any side tubing of it will cease, again indicating a correct position of the reinforcement 16 around and outside of the opening 51 of the therapeutic sheath 50.

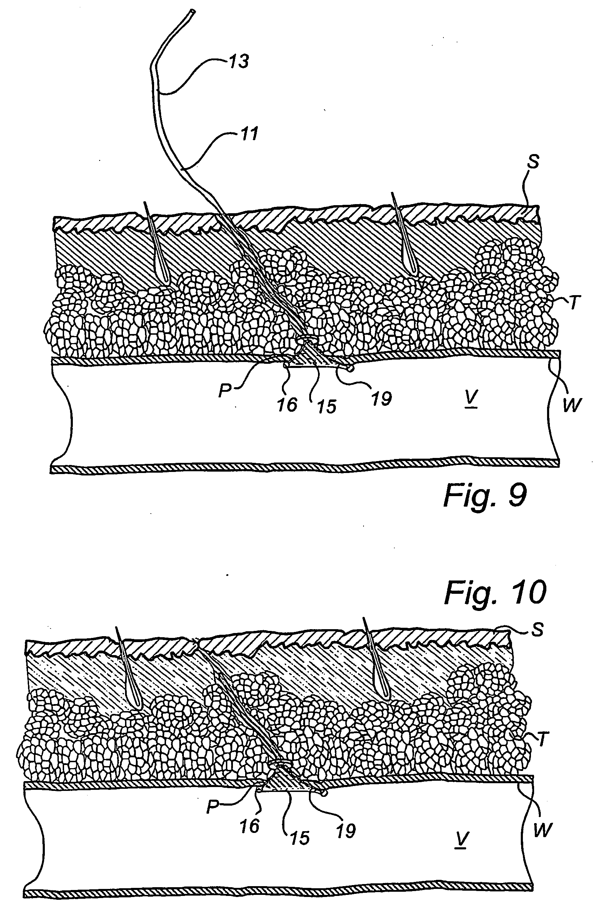

As illustrated in Fig. 8, the sheath 50 is removed and the expanded reinforcement 16 is brought into its final and correct position in the artery 7, the blood flow B being still present. As schematically illustrated in Fig. 8 in comparison with Fig. 9, the portion 19 of the tubing adjacent the reinforcement has, however, still not reached its final shape. The indicative blood flows out through the passageway 14. In case the blood flow ceases, this would be an indication that the reinforcement incorrectly has been pulled out from the vessel V.

Having obtained the position in Fig. 8, the sealing member 10 is ready to be pursed. In the present embodiment, this is performed by applying the pursing member 20 around the proximal end 13 of the tubing 11 that is extending outside the patient either over the end 13 or from the side of the tubing 11. The pursing member 20 is then advanced along the tubing 11, through the skin S and as close to the artery V as possible. This results in that the distal region 12 of the tubing 11 receives a funnel-shape as indicated at 19 in Fig. 9. Thus, there will be created an inwardly and upwardly directed "pulling force" F in the wall of the tubing in the region 19. Due to the elastic force of the reinforcement 16, the region 12 will not collapse in the plane of the distal opening 15. However, the pulling force F will "lift" the reinforcement and press the reinforced distal end region 12 against the inner wall of the artery 7 to form a tight sealing of the puncture P, as shown at reference numeral 19 in Fig. 9.

It should be noted that the thus-applied pursing member 20 should preferably be held in place in relation to the body, since it may otherwise move towards the body vessel V, whereby the above-described sealing effect may be lost. This is preferably achieved by a direct engagement between exterior of the pursing member 20 and the body tissue T. A less preferred embodiment would include applying some kind of maintaining force on the proximal part of the tubing 11.

Finally, the extending proximal end or region 13 of the tubing 11 is cut by a knife or by scissors at the skin level. This is preferably performed with a light traction of the tubing 11 that makes the remains of the tubing 11 spring back and retract under the skin S, as shown in Fig. 10.

It should be noted that Figs 8-10 also show the structure of the tissue T between the artery 7 and the skin layer S, this tissue T usually comprising fat cells.

The described method of sealing an artery puncture P is very advantageous. The patient can ambulate immediately and leave the hospital without the need of lying in bed. The risk of hematomas is minimized. Furthermore there is no need of suturing.

The device and the method of its use have been described in view of an artery puncture, but it should be obvious that the device and the method can be used for sealing body vessels in general, for both human and animal bodies. The body vessel may be e.g. a blood vessel, bile duct, urinary duct or gastrointestinal tract.

The remaining hole in the skin is covered by a plaster to heal. The sealing member 10 and the pursing member 20 that are left inside the body are resorbed. Using the device is very easy and there is no need for suturing to fasten,the sealing member 10. Furthermore the discomfort to the patient is minimal.

The present invention may be used within nearly any catheterization or other medical procedures such as laparoscopic or other minimally or less invasive surgeries wherein it is desirable to seal an incision or a puncture in the patient to prevent the loss of the patient's body fluid there through.

## Claims

1. An apparatus for sealing a puncture (P) in a body vessel (V), said apparatus including:
a sealing member (10) comprising a flexible tubing (11) having an internal passageway (14) and
a pursing member (20) arranged to purse said flexible tubing (11) in order to seal said passageway (14), **characterised in that**
said flexible tubing (11) has an open distal end, and that said tubing (11) at said open distal end (12) is provided with an elastic expansion member (16) for self-expanding said open distal end (12) to an expanded configuration when said elastic expansion member (16) has been introduced into the vessel (V), such that the tubing (11) at the distal open end (12) thereafter may be brought into sealing contact with an inner wall (W) of the vessel (V).

2. An apparatus as claimed in claim 1, wherein the flexible tubing (11) presents a sufficient length for the tubing, when positioned in the body, to extend from said puncture (P) to the outside of the body.

3. An apparatus as claimed in claim 1 or 2, wherein said flexible tubing (11), when being in a non-compressed, straight configuration, presents an essentially constant cross-section along its length.

4. An apparatus as claimed in claim 1 or 2, wherein said flexible tubing (11), when being in a non-compressed, straight configuration, presents a cross-section that tapers proximally from the distal end (12).

5. An apparatus as claimed in any one of the preceding claims, wherein said elastic member (16) is foldable to a non-expanded configuration, allowing said elastic member (16) to be introduced through the puncture (P), and unfoldable in a self-expandable manner to said expanded configuration.

6. An apparatus as claimed in any one of the preceding claims, wherein said elastic member (16) is flexible in its expanded configuration to such an extent that its shape is adjustable to the curvature of an inner wall (W) of the body vessel (V).

7. An apparatus as claimed in any one of the preceding claims, wherein said elastic member (16) is ring-shaped when in its expanded configuration.

8. An apparatus as claimed in any one of the preceding claims, wherein said elastic member (16) is integrally formed with the flexible tubing (11) at the distal open end (12) thereof.

9. An apparatus as claimed in any one of the preceding claims, wherein the material of said flexible tubing (11) and said elastic member (16) is selected from a group consisting of a synthetic medical plastic material like polyurethane, polyvinyl, polytetrafluorethylene (PTFE) or other polymers, and a biologically degradable material like polydioxanone (PDS), polyglactin (Vicryl), polyglycolic acid (Dexon) or other resorbable materials that slowly will be absorbed by the body.

10. An apparatus as claimed in any one of the preceding claims, wherein said elastic member (16) is integrally formed with the flexible tubing (11) in the form of a reinforcement of said tubing (11) at the distal opening (12) thereof.

11. An apparatus as claimed in any one of claims 1-9, wherein said elastic member (16) is integrally formed with the flexible tubing (11) in the form of a material thickening of said tubing (11) at the distal opening (12) thereof.

12. An apparatus as claimed in any one of claims 1-9, wherein said elastic member (16) is integrally formed with the flexible tubing (11) in the form of a stiffening means attached to said tubing (11) at the distal opening (12) thereof.

13. An apparatus as claimed in any one of the preceding claims, wherein said elastic member (16) includes an X-ray absorbing material.

14. An apparatus as claimed in any one of the preceding claims, wherein said pursing member (20) is arranged to be positioned in a final sealing position adjacent said puncture (P) for sealing said passageway (14).

15. An apparatus as claimed in any one of the preceding claims, wherein said pursing member (20) is formed as a member separate from said flexible tubing (11).

16. An apparatus as claimed in any one of the preceding claims, wherein said pursing member (20) is arranged to be positioned on said flexible tubing (11) for sealing the passageway (14) after said elastic member (16) has self-expanded to its expanded configuration and been brought into contact with said inside wall (W) of the vessel (V) at said puncture (P).

17. An apparatus as claimed in any one of the preceding claims, wherein said pursing member (20) is a ring-shaped pursing member (20), which is arranged to circumferentially purse said flexible tubing (11) in order to seal said passageway (14).

18. An apparatus as claimed in claim 17, wherein said ring-shaped pursing member (20), when arranged on and circumferentially pursing said flexible tubing (11), is displaceable along the flexible tubing (11).

19. An apparatus as claimed in any one of claims 1-16, wherein said pursing member (20) is a clip.

20. An apparatus as claimed in any one of the preceding claims, wherein the material of said pursing member (20) is selected from a group consisting of a synthetic medical plastic material like polyurethane, polyvinyl, polytetrafluorethylene (PTFE) or other polymers, and a biological degradable material like polydioxanone (PDS), polyglactin (Vicryl), polyglycolic acid (Dexon) or other resorbable materials that slowly will be absorbed by the body.

21. An apparatus as claimed in any one of the preceding claims, wherein said pursing member (20), when occupying a final position in which it purses said flexible tubing (11) and thereby seals said passageway (14), is arranged to also maintain the expanded distal end (12) of the flexible tubing (11) in a sealed engagement with the inner wall (W) of the body vessel (V).

22. An apparatus as claimed in any one of the preceding claims, wherein said flexible tubing (11) is flexible in the sense that it has essentially no ability to maintain its shape by its own.

23. An apparatus as claimed in any one of the preceding claims, further comprising an insertion tube (30) having a central bore or channel, which is arranged to initially receive the elastic member (16) in a non-expanded configuration and at least part of the flexible tubing (11).

24. An apparatus as claimed in claim 23, wherein the elastic member (16) and at least part of the flexible tubing (11) are initially arranged inside the insertion tube (30) before use of the apparatus.

25. An apparatus as claimed in claim 23 or 24, wherein the insertion tube (30) is pliable.

26. An apparatus as claimed in any one of the preceding claims, further comprising a pushing device (40), such as a solid rod or a tube, for pushing the elastic member (16) through said puncture (P) and into said body vessel (V).

## Patentansprüche

1. Vorrichtung zum Verschließen einer Punktionsstelle (P) in einem Körpergefäß (V), wobei die Vorrichtung Folgendes enthält:
ein Verschlusselement (10), das einen flexiblen Schlauch (11) umfasst, der einen inneren Durchgang (14) aufweist; und
ein Abschnürelement (20), das dafür ausgelegt ist, den flexiblen Schlauch (11) abzuschnüren, um den Durchgang (14) zu verschließen,
**dadurch gekennzeichnet, dass** der flexible Schlauch (11) einen inneren Durchgang (14) und ein offenes distales Ende aufweist, und dass der Schlauch (11) an dem offenen distalen Ende (12) mit einem elastischen Aufweitungselement (16) versehen ist, welches das offene distale Ende (12) selbsttätig in einen aufgeweiteten Zustand aufweitet, wenn das elastische Aufweitungselement (16) in das Gefäß (V) eingeführt wurde, dergestalt, dass der Schlauch (11) am distalen offenen Ende (12) anschließend in einen abdichtenden Kontakt mit einer Innenwand (W) des Gefäßes (V) gebracht werden kann.

2. Vorrichtung nach Anspruch 1, wobei der flexible Schlauch (11) eine ausreichende Länge aufweist, damit der Schlauch, wenn er in dem Körper positioniert ist, von der Punktionsstelle (P) bis nach außerhalb des Körpers reicht.

3. Vorrichtung nach Anspruch 1 oder 2, wobei der flexible Schlauch (11), wenn er sich in einem nicht zusammengedrückten, geraden Zustand befindet, einen im Wesentlichen konstanten Querschnitt entlang seiner Länge aufweist.

4. Vorrichtung nach Anspruch 1 oder 2, wobei der flexible Schlauch (11), wenn er sich in einem nicht zusammengedrückten, geraden Zustand befindet, einen Querschnitt aufweist, der sich proximal von dem distalen Ende (12) verjüngt.

5. Vorrichtung nach einem der vorangehenden Ansprüche, wobei das elastische Element (16) in einen nicht aufgeweiteten Zustand zusammengelegt werden kann, wodurch das elastische Element (16) durch die Punktionsstelle (P) hindurch eingeführt werden kann, und in einer selbst-aufweitenden Weise in den aufgeweiteten Zustand aufgeklappt werden kann.

6. Vorrichtung nach einem der vorangehenden Ansprüche, wobei das elastische Element (16) in seinem aufgeweiteten Zustand in einem solchen Ausmaß flexibel ist, dass seine Form an die Krümmung einer Innenwand (W) des Körpergefäßes (V) anpassbar ist.

7. Vorrichtung nach einem der vorangehenden Ansprüche, wobei das elastische Element (16) ringförmig ist, wenn es sich in seinem aufgeweiteten Zustand befindet.

8. Vorrichtung nach einem der vorangehenden Ansprüche, wobei das elastische Element (16) integral mit dem flexiblen Schlauch (11) an seinem distalen offenen Ende (12) ausgebildet ist.

9. Vorrichtung nach einem der vorangehenden Ansprüche, wobei das Material des flexiblen Schlauchs (11) und des elastischen Elements (16) aus einer Gruppe ausgewählt ist, die aus einem synthetischen medizinischen Kunststoffmaterial wie Polyurethan, Polyvinyl, Polytetrafluorethylen (PTFE) oder anderen Polymeren und einem biologisch abbaubaren Material wie Polydioxanon (PDS), Polyglactin (Vicryl), Polyglycolsäure (Dexon) oder anderen resorbierbaren Materialien, die langsam vom Körper absorbiert werden, besteht.

10. Vorrichtung nach einem der vorangehenden Ansprüche, wobei das elastische Element (16) integral mit dem flexiblen Schlauch (11) in der Form einer Verstärkung des Schlauchs (11) an seiner distalen Öffnung (12) ausgebildet ist.

11. Vorrichtung nach einem der Ansprüche 1-9, wobei das elastische Element (16) integral mit dem flexiblen Schlauch (11) in der Form einer Materialverdickung des Schlauchs (11) an seiner distalen Öffnung (12) ausgebildet ist.

12. Vorrichtung nach einem der Ansprüche 1-9, wobei das elastische Element (16) integral mit dem flexiblen Schlauch (11) in der Form eines Versteifungsmittels, das an dem Schlauch (11) an seiner distalen Öffnung (12) angebracht ist, ausgebildet ist.

13. Vorrichtung nach einem der vorangehenden Ansprüche, wobei das elastische Element (16) ein Röntgenstrahlen absorbierendes Material ist.

14. Vorrichtung nach einem der vorangehenden Ansprüche, wobei das Abschnürelement (20) dafür ausgelegt ist, in einer endgültigen Verschlussposition neben der Punktionsstelle (P) zum Verschließen des Durchgangs (14) angeordnet zu werden.

15. Vorrichtung nach einem der vorangehenden Ansprüche, wobei das Abschnürelement (20) als ein von dem flexiblen Schlauch (11) separates Element ausgebildet ist.

16. Vorrichtung nach einem der vorangehenden Ansprüche, wobei das Abschnürelement (20) dafür ausgelegt ist, an dem flexiblen Schlauch (11) zum Verschließen des Durchgangs (14) angeordnet zu werden, nachdem sich das elastische Element (16) selbsttätig in seinen aufgeweiteten Zustand aufgeweitet hat und mit der Innenwand (W) des Gefäßes (V) an der Punktionsstelle (P) in Kontakt gebracht wurde.

17. Vorrichtung nach einem der vorangehenden Ansprüche, wobei das Abschnürelement (20) ein ringförmiges Abschnürelement (20) ist, das dafür ausgelegt ist, den flexiblen Schlauch (11) umfänglich abzuschnüren, um den Durchgang (14) zu verschließen.

18. Vorrichtung nach Anspruch 17, wobei das ringförmige Abschnürelement (20), wenn es an dem flexiblen Schlauch (11) angeordnet ist und diesen umfänglich abschnürt, entlang des flexiblen Schlauchs (11) verschoben werden kann.

19. Vorrichtung nach einem der Ansprüche 1-16, wobei das Abschnürelement (20) eine Klammer ist.

20. Vorrichtung nach einem der vorangehenden Ansprüche, wobei das Material des Abschnürelements (20) aus einer Gruppe ausgewählt ist, die aus einem synthetischen medizinischen Kunststoffmaterial wie Polyurethan, Polyvinyl, Polytetrafluorethylen (PTFE) oder anderen Polymeren und einem biologisch abbaubaren Material wie Polydioxanon (PDS), Polyglactin (Vicryl), Polyglycolsäure (Dexon) oder anderen resorbierbaren Materialien, die langsam vom Körper absorbiert werden, besteht.

21. Vorrichtung nach einem der vorangehenden Ansprüche, wobei das Abschnürelement (20), wenn es eine endgültige Position eingenommen hat, in der es den flexiblen Schlauch (11) abschnürt und **dadurch** den Durchgang (14) verschließt, dafür ausgelegt ist, außerdem das aufgeweitete distale Ende (12) des flexiblen Schlauchs (11) in einem abdichtenden Eingriff mit der Innenwand (W) des Körpergefäßes (V) zu halten.

22. Vorrichtung nach einem der vorangehenden Ansprüche, wobei der flexible Schlauch (11) in dem Sinne flexibel ist, dass er im Wesentlichen nicht in der Lage ist, seine Form von allein beizubehalten.

23. Vorrichtung nach einem der vorangehenden Ansprüche, die des Weiteren ein Einführröhrchen (30) umfasst, das eine mittige Bohrung oder einen mittigen Kanal aufweist und das dafür ausgelegt ist, zunächst das elastische Element (16) in einem nicht aufgeweiteten Zustand und mindestens einen Teil des flexiblen Schlauchs (11) aufzunehmen.

24. Vorrichtung nach Anspruch 23, wobei das elastische Element (16) und mindestens ein Teil des flexiblen Schlauchs (11) anfänglich im Inneren des Einführröhrchens (30) angeordnet sind, bevor die Vorrichtung verwendet wird.

25. Vorrichtung nach Anspruch 23 oder 24, wobei das Einführröhrchen (30) biegsam ist.

26. Vorrichtung nach einem der vorangehenden Ansprüche, die des Weiteren eine Schiebevorrichtung (40), wie zum Beispiel einen massiven Stab oder eine Röhre, umfasst, um das elastische Element (16) durch die Punktionsstelle (P) hindurch und in das Körpergefäß (V) hinein zu schieben.

## Revendications

1. Dispositif pour l'obturation d'une perforation (P) dans un vaisseau corporel (V), ledit dispositif incluant:
un élément d'obturation (10) comprenant une tubulure flexible (11) possédant un passage interne (14) et
un élément de froncement (20) agencé afin de froncer ladite tubulure flexible (11) de manière à obturer ledit passage (14), **caractérisé en ce que** ladite tubulure flexible (11) possède une extrémité distale ouverte et que ladite tubulure (11) est pourvue au niveau de ladite extrémité distale ouverte (12) d'un élément d'expansion élastique (16) à des fins d'auto-expansion de ladite extrémité distale ouverte (12) en une configuration expansée quand ledit élément d'expansion élastique (16) a été introduit à l'intérieur du vaisseau (V), de sorte que la tubulure (11) au niveau de l'extrémité distale ouverte (12) puisse ensuite être amenée en contact obturateur avec une paroi interne (W) du vaisseau (V).

2. Dispositif selon la revendication 1, dans lequel la tubulure flexible (11) présente une longueur suffisante pour que la tubulure, quand elle est positionnée dans le corps, s'étende à partir de ladite perforation (P) vers l'extérieur du corps.

3. Dispositif selon la revendication 1 ou 2, dans lequel ladite tubulure flexible (11), quand elle se trouve dans une configuration non comprimée, droite, présente une section transversale essentiellement constante le long de sa longueur.

4. Dispositif selon la revendication 1 ou 2, dans lequel ladite tubulure flexible (11), quand elle se trouve dans une configuration non comprimée, droite, présente une section transversale qui s'effile proximalement à partir de l'extrémité distale (12).

5. Dispositif selon une quelconque des revendications précédentes, dans lequel ledit élément élastique (16) est pliable en une configuration non expansée, permettant audit élément élastique (16) d'être introduit à travers la perforation (P), et impliable d'une manière non expansible en ladite configuration expansée.

6. Dispositif selon une quelconque des revendications précédentes, dans lequel ledit élément élastique (16) est flexible dans sa configuration expansée dans une proportion telle que sa forme puisse être ajustée sur l'incurvation d'une paroi interne (W) du vaisseau corporel (V).

7. Dispositif selon une quelconque des revendications précédentes, dans lequel ledit élément élastique (16) est de forme annulaire quand il se trouve dans sa configuration expansée.

8. Dispositif selon une quelconque des revendications précédentes, dans lequel ledit élément élastique (16) est façonné en un seul tenant avec la tubulure flexible (11) à l'extrémité distale ouverte (12) de celle-ci.

9. Dispositif selon une quelconque des revendications précédentes, dans lequel le matériau de ladite tubulure flexible (11) et dudit élément élastique (16) est sélectionné dans un groupe consistant en un matériau de plastique médical synthétique, comme du polyuréthane, du polyvinyle, du polytétrafluoréthylène (PTFE) ou d'autres polymères, et un matériau biologiquement dégradable comme du polydioxanone (PDS), de la polyglactine (vicryle), de l'acide polyglycolique (Dexon) ou d'autres matériaux résorbables qui seront lentement absorbés par le corps.

10. Dispositif selon une quelconque des revendications précédents, dans lequel ledit élément élastique (16) est façonné en un seul tenant avec la tubulure flexible (11) sous la forme d'un renforcement de ladite tubulure (11) à l'ouverture distale (12) de celle-ci.

11. Dispositif selon une quelconque des revendications 1 à 9, dans lequel ledit élément élastique (16) est façonné en un seul tenant avec la tubulure flexible (11) sous la forme d'un épaississement de matériau de ladite tubulure (11) à l'ouverture distale (12) de celle-ci.

12. Dispositif selon une quelconque des revendications 1 à 9, dans lequel ledit élément élastique (16) est façonné en une seul tenant avec la tubulure flexible (11) sous la forme d'un moyen de raidissement fixé à ladite tubulure (11) à l'ouverture distale (12) de celle-ci.

13. Dispositif selon une quelconque des revendications précédentes, dans lequel ledit élément élastique (16) inclut un matériau absorbant de rayons X.

14. Dispositif selon une quelconque des revendications précédentes, dans lequel ledit élément de froncement (20) est agencé afin d'être positionné dans une position d'obturation finale adjacente à ladite perforation (P) pour obturer ledit passage (14).

15. Dispositif selon une quelconque des revendications précédentes, dans lequel ledit élément de froncement (20) est façonné comme un élément séparé de ladite tubulure flexible (11).

16. Dispositif selon une quelconque des revendications précédentes, dans lequel ledit élément de froncement (20) est agencé afin d'être positionné sur ladite tubulure flexible (11) pour obturer le passage (14) après que ledit élément élastique (16) s'est auto-expansé en sa configuration expansée et a été amené en contact avec ladite paroi interne (W) du vaisseau (V) au niveau de ladite perforation (P).

17. Dispositif selon une quelconque des revendications précédentes, dans lequel ledit élément de froncement (20) est un élément de froncement (20) de forme annulaire, qui est agencé afin de froncer circonférentiellement ladite tubulure flexible (11) de manière à obturer ledit passage (14).

18. Dispositif selon la revendication 17, dans lequel ledit élément de froncement (20) de forme annulaire, quand il est agencé sur et fronce circonférentiellement ladite tubulure flexible (11), est déplaçable le long de la tubulure flexible (11).

19. Dispositif selon une quelconque des revendications 1 à 16, dans lequel ledit élément de froncement (20) est une fixation à ressort.

20. Dispositif selon une quelconque des revendications précédentes, dans lequel le matériau dudit élément de froncement (20) est sélectionné dans un groupe consistant en un matériau de plastique médical synthétique comme du polyuréthane, du polyvinyle, du polytétrafluoréthylène (PTFE) ou d'autres polymères, et un matériau biologiquement dégradable comme du polydioxanone (PDS), de la polyglactine (vicryle), de l'acide polyglycolique (Dexon) ou d'autres matériaux résorbables qui seront lentement absorbés par le corps.

21. Dispositif selon une quelconque des revendications précédentes, dans lequel ledit élément de froncement (20), quand il occupe une position finale dans laquelle il fronce ladite tubulure flexible (11) et obture ainsi ledit passage (14), est agencé afin de maintenir aussi l'extrémité distale expansée (12) de la tubulure flexible (11) en mise en prise obturée avec la paroi interne (W) du vaisseau corporel (V).

22. Dispositif selon une quelconque des revendications précédentes, dans lequel ladite tubulure flexible (11) est flexible dans le sens qu'elle n'a essentiellement pas d'aptitude à conserver sa forme par elle-même.

23. Dispositif selon une quelconque des revendications précédentes, comprenant en outre un tube d'insertion (30) possédant un alésage ou canal central, qui est agencé afin de recevoir initialement l'élément élastique (16) dans une configuration non expansée et au moins une partie de la tubulure flexible (11).

24. Dispositif selon la revendication 23, dans lequel l'élément élastique (16) et au moins une partie de la tubulure flexible (11) sont initialement agencés à l'intérieur du tube d'insertion (30) avant l'utilisation de l'appareil.

25. Dispositif selon la revendication 23 ou 24, dans lequel le tube d'insertion (30) est pliable.

26. Dispositif selon une quelconque des revendications précédentes, comprenant en outre un dispositif de poussée (40), comme une tige ou un tube solide, pour pousser l'élément élastique (16) à travers ladite perforation (P) et à l'intérieur dudit vaisseau corporel (V).
